# EUROPÄISCHE PATENTSCHRIFT

(11) **EP 2 190 369 B1**
(45) Veröffentlichungstag und Bekanntmachung des Hinweises auf die Patenterteilung: **20.07.2016**
(21) Anmeldenummer: 08804839.2
(22) Anmeldetag: 26.09.2008
(51) Int. Cl.: A61B 17/86, A61B 17/80, A61B 17/74

(54) **KNOCHENFIXIERUNGSVORRICHTUNG ZUR OPERATIVEN VERSORGUNG VON FRAKTUREN**
BONE ANCHORING DEVICE FOR THE OPERATIVE REPAIR OF FRACTURES
DISPOSITIF DE FIXATION D'OS EN POSITION POUR LE TRAITEMENT OPÉRATOIRE DE FRACTURES

(30) Priorität: 26.09.2007 EP 07117227; 19.12.2007 CH 19922007
(43) Veröffentlichungstag der Anmeldung: 02.06.2010
(73) Patentinhaber: Zimmer GmbH, 8404 Winterthur (CH); Kuster, Markus, 9000 St Gallen (CH)
(72) Erfinder: KUSTER, Markus, CH-9000 St Gallen (CH); VELIKOV, Jordan, 8044 Gockhausen (CH); GARIC, Miodrag, CH-8404 Winterthur (CH); TESCHKE, Thomas, CH-8049 Zürich (CH)
(74) Vertreter: Manitz, Finsterwald & Partner GbR
(86) Internationale Anmeldenummer: PCT/EP2008/062974
(87) Internationale Veröffentlichungsnummer: WO 2009/043827

(56) Entgegenhaltungen:
- EP-A- 0 507 162
- WO-A-01/91660
- WO-A-03/101321
- WO-A-2004/075766
- US-A- 5 269 784
- US-A1- 2005 234 457

## Beschreibung

### Technisches Gebiet

Die Erfindung betrifft eine Knochenfixierungsvorrichtung der in Anspruch 1 beschriebenen Art.

### Stand der Technik

Zur Behandlung einer Knochenfraktur wie beispielsweise einer proximalen Humerusfraktur wird die Fraktur nach Ausrichtung der einzelnen Knochenfragmente in ihre korrekte Lage (Reposition) mittels Stiften, Schrauben, Drähten oder Platten fixiert. Insbesondere kann zur Fixierung eine Platte eingesetzt werden, die mittels Schrauben an den einzelnen Knochenfragmenten befestigt ist. Bei dieser Art der Behandlung wird eine Kompressionswirkung erzielt, derart, dass die einzelnen Knochenfragmente durch die Platte und die Schrauben in einer festen Position zueinander gehalten und aneinander gedrückt werden. Um das Risiko einer periostalen Durchblutungsstörung zu reduzieren, wird die zur Fixierung eingesetzte Platte beispielsweise in einem Abstand, d.h. ohne Kontakt zur Knochenoberfläche, fixiert.

Von der Anmelderin wird beispielsweise zur Versorgung von Frakturen des proximalen Humerus unter der Bezeichnung NCB (Non-Contact Bridging) eine Osteosyntheseplatte angeboten, die eine polyaxiale Schraubenanordnung mit anschließender Schraubensicherung für höhere Stabilität, besonders in porotischen Knochen, erlaubt. Die Schrauben können in der Platte zur optimalen Versorgung der Fraktur winkelstabil unter variierenden Winkeln bis ca. 30° fixiert werden. Nach der geeigneten Positionierung einer Schraube wird diese in der Platte fest geklemmt, um die Frakturfragmente in der korrekten Position zueinander zu fixieren. Im gesicherten Zustand dient daher die Osteosyntheseplatte als interner Fixateur für die Humerusfräktur ohne Kontakt der Platte zur Knochenoberfläche. Hierdurch wird das Risiko einer periostalen Durchblutungsstörung reduziert.

Die US 2005/0234457 A1 offenbart eine Knochenfixierungsvorrichtung nach dem Oberbegriff des Anspruchs 1.

### Darstellung der Erfindung

Hier wird nun eine Knochenfixierungsvorrichtung zur operativen Versorgung von Frakturen gemäß den Merkmalen des Anspruchs 1 vorgeschlagen, bei welcher bei Verwendung in einem gelenksnahen Bereich das Risiko einer Verletzung des Gelenks verringert ist, und/oder bei welcher die Kompressionswirkung verringert ist.

Es ist eine Knochenfixierungsvorrichtung zur operativen Versorgung von Frakturen vorgesehen. Die Knochenfixierungsvorrichtung umfasst eine Frakturfixierungsplatte, wobei die Platte wenigstens eine Öffnung aufweist. Insbesondere weist die Platte in einer Ausgestaltung eine zur Anordnung vom Knochen abgewandt ausgebildete erste Seite und eine zur Anordnung zum Knochen hin ausgebildete zweite Seite auf, wobei in der Platte wenigstens eine von der ersten zur zweiten Seite führende Öffnung angeordnet ist. Die zur Anordnung zum Knochen hin ausgebildete Seite der Platte zeichnet sich insbesondere dadurch aus, dass deren Formgebung diesem Verwendungszweck angepasst ist. Die Geometrie und Kontur dieser Seite ist derart ausgestaltet, dass die Plätte an dieser Seite im Wesentlichen eine typische Negativform des Knochens aufweist, an dem sie zur Anordnung vorgesehen ist. Das heisst, diese Form wird nicht von Patient zu Patient angepasst, sondern ist einem typischen "Durchschnitts"knochen angepasst, wobei eine Platte für einen Knochen üblicherweise in mehreren Grössen vorliegt. Die Knochenfixierungsvorrichtung umfasst ferner wenigstens ein Verankerungselement zur Befestigung der Fraktufixierungsplatte am Knochen, wobei das Verankerungselement einen Schaft sowie einen Kopf aufweist. Bevorzugt ist der Kopf an einem manipulationsseitigen Ende des Verankerungselements angeordnet. Wenigstens ein Teil des Schafts ist durch die Öffnung hindurchführbar, während der Kopf in der Öffnung fixierbar ist. Der Schaft ist wenigstens zweiteilig, insbesondere zweiteilig, ausgeführt, mit einem ersten Teil, an dem der Kopf angeordnet ist, und mit einem zweiten zur Verankerung im Knochen vorgesehenen Teil. Im am Knochen fixierten Zustand der Knochenfixierungsvorrichtung sind der erste Teil und der zweite Teil entlang einer Achse zueinander verschiebbar geführt, und sind insbesondere frei verschiebbar geführt. Gemäß einer Ausführungsform können der erste Teil und der zweite Teil lineargeführt in Richtung der Achse zueinander gleiten, insbesondere frei gleiten. Gemäß einer Ausführungsform ist der Schaft insbesondere teleskopisch ausziehbar, ausfahrbar und/oder verlängerbar.

Ein wesentlicher Gedanke von Ausführungsformen der Erfindung besteht darin, bei einer Knochenfixierungsvorrichtung eine Frakturfixierungsplatte zur Fixierung von Knochenfragmenten einzusetzen, die mittels zumindest eines Verankerungselements oder einer Gleitverankerung, insbesondere gebildet aus einer Hülse und einem darin gleitend gelagerten stiftförmigen Element oder Befestigungselement, am Knochen fixiert wird, wobei im am Knochen fixierten Zustand der Knochenfixierungsvorrichtung, insbesondere nicht nur beim Zusammensetzen sondern auch noch im am Knochen fixierten Zustand, der erste Teil und der zweite Teil des Schafts des jeweiligen Verankerungselements zueinander verschiebbar geführt sind.

Beispielsweise kann das Verankerungselement dazu verwendet werden, zwei Knochenfragmente einer Gelenkkopffraktur aneinander zu halten, deren Frakturlinie quer zu der Achse des Verankerungselements verläuft. Sofern sich in einem derartigen Fall das eine, insbesondere in Kontakt mit der zughörigen Gelenkpfanne befindliche Knochenfragment in Richtung des anderen Knochenfragments bewegt, beispielsweise durch Zusammensintern, insbesondere während des Heilungsprozesses, kann ein Durchstoßen des Verankerungselements in das Gelenk verhindert werden. Dies ergibt sich daraus, dass die Spitze des Verankerungselements, die in dem einen Knochenfragment verankert ist, aufgrund der Verschiebbarkeit der beiden Schaftteile zueinander mit dem einen Knochenfragment mitwandern kann. Vorteilhaft kann das Verankerungselement auch bei Frakturen von Knochen von Kindern oder Jugendlichen mit einer quer zu der Achse des Verankerungselements verlaufenden Wachstumslinie eingesetzt werden. Durch die Verschiebbarkeit der beiden Schaftteile zueinander, wird ein Knochenwachstum möglichst wenig behindert.

Durch die Verwendung eines derartigen Verankerungselements kann die Kompressionswirkung der Knochenfixierungsvorrichtung wirksam reduziert werden, da im Wesentlichen nur Querkräfte durch das Verankerungselement übertragen werden, nicht aber längs des Verankerungselements wirkende Kräfte.

Dadurch kann insbesondere im gelenksnahen Bereich einer möglichen Verletzung der Gelenkfläche aufgrund der reduzierten Kompressionswirkung vorgebeugt werden, da sich das Verankerungselement bei Belastung des Gelenks in Richtung der Achse, insbesondere seiner Längsachse, und damit zur Platte verschieben und damit unter Umständen ein Durchstoßen des Verankerungselements in das Gelenk verhindert werden kann.

Unter einem Verankerungselement ist im Rahmen der vorliegenden Erfindung jede Art von Stabilisierungs- oder Verankerungselement zu verstehen, das mit einer Knochenfixierungsvorrichtung verwendet werden kann, also insbesondere auch Gleitstangen oder -bolzen in Kombination mit Hülsen, in denen sie gleitend gelagert sind, wobei die Hülsen im Knochen verankert sein können.

Gemäß einer Ausführungsform umfasst das Verankerungselement eine Hülse mit einem Innenführungsbereich und ein darin aufgenommenes, insbesondere stiftförmiges Element mit einem Außenführungsbereich, welche Bereiche in Richtung der Achse jeweils einen konstanten Querschnitt aufweisen, derart, dass der Außenführungsbereich im Innenführungsbereich lineargeführt in Richtung der Achse gleiten kann. Die Querschnitte von Innenführungsbereich und Aussenführungsbereich sind aufeinander abgestimmt, um ein Gleiten ineinander zu ermöglichen, und sind in einer Ausführungsform kreisförmig.

Gemäß einer Ausführungsform ist eine Knochenfixierungsvorrichtung zur operativen Versorgung von Frakturen vorgesehen, die folgendes umfasst:
- mindestens eine Hülse, die zur gleitenden Aufnahme eines stiftförmigen Befestigungselements derart ausgebildet ist, dass das stiftförmige Befestigungselement entlang seiner Längsachse in der Hülse gleiten kann,
- mindestens ein stiftförmiges Befestigungselement, das einen Verankerungsabschnitt zur Fixierung des Elements und einen Gleitabschnitt für die gleitende Aufnahme in der Hülse aufweist, und
- eine Frakturfixierungsplatte, die mindestens eine Öffnung zur Lagerung und/oder Fixierung der aus einer Hülse und einem gleitend darin gelagerten stiftförmigen Befestigungselement gebildeten Gleitverankerung in einem bestimmten Winkel zur Frakturfixierungsplatte aufweist.
Bei einer derartigen Knochenfixierungsvorrichtung kann die Gleitverankerung entlang seiner Längsachse in der Hülse gleiten, so dass bei einer beispielsweise durch eine Muskelkontraktion bewirkten Belastung der Fraktur insbesondere des proximalen Humerus effizient einem Durchstoßen des Knochens durch die Spitze der Gleitverankerung vorgebeugt werden kann.

Gemäß einer Ausführungsform ist das insbesondere stiftförmige Element zur Verankerung im Knochen und die Hülse zur Fixierung in der wenigstens einen Öffnung der Frakturfixierungsplatte ausgebildet. Insbesondere kann das stiftförmige Element oder Befestigungselement eine Knochenschraube sein und kann der vorgenannte Verankerungsabschnitt der Knochenschraube zur Fixierung der Schraube in einem Knochen ausgebildet sein, beispielsweise ein Gewinde zum Einschrauben in die Spongiosa eines Knochens aufweisen, und die mindestens eine Öffnung der Frakturfixierungsplatte kann zur Lagerung und/oder Fixierung der Hülse in einem bestimmten Winkel zur Frakturfixierungsplatte ausgebildet sein. Bei dieser Ausführungsform wird also die Hülse in der Frakturfixierungsplatte gelagert und/oder fixiert, während die Knochenschraube als stiftförmiges Element gleitend in der Hülse gelagert und im Knochen verankert wird.

Gemäß einer anderen Ausführungsform ist die Hülse zur Verankerung im Knochen und das insbesondere stiftförmige Element zur Fixierung in der wenigstens einen Öffnung der Frakturfixierungsplatte ausgebildet. Insbesondere kann das stiftförmige Element oder Befestigungselement ein Gleitstift sein und kann der vorgenannte Verankerungsabschnitt des Gleitstifts zur Fixierung des Gleitstifts in der mindestens einen Öffnung der Frakturfixierungsplatte ausgebildet sein, und die mindestens eine Öffnung der Frakturfixierungsplatte kann zur Lagerung und/oder Fixierung des Gleitstifts in einem bestimmten Winkel zur Frakturfixierungsplatte ausgebildet sein. Hier wird also die Hülse im Knochen verankert, beispielsweise eingeschraubt, und der Gleitstift als stiftförmiges Element gleitend in der im Knochen verankerten Hülse gelagert und in der Öffnung der Frakturfixierungsvorrichtung fixiert.

Gemäß einer Ausführungsform ist eine Knochenfixierungsvorrichtung mit einer wenigstens zwei Öffnungen aufweisenden Frakturfixierungsplatte und mit wenigstens zwei Verankerungselementen vorgesehen, wobei wenigstens ein Verankerungselement derart ausgebildet ist, dass das stiftförmige Element zur Verankerung im Knochen und die Hülse zur Fixierung in der wenigstens einen Öffnung der Frakturfixierungsplatte ausgebildet ist, und wobei wenigstens ein anderes Verankerungselemente derart ausgebildet ist, dass die Hülse zur Verankerung im Knochen und das insbesondere stiftförmige Element zur Fixierung in der wenigstens einen Öffnung der Frakturfixierungsplatte ausgebildet ist. In weiteren Ausführungsformen sind wenigstens ein wie beschrieben gleitendes längenvariables und ein längeninvariables Verankerungselement in der Platte angeordnet oder zur Anordnung in der Platte vorgesehen.

Die Hülse oder der Gleitstift kann den Kopf oder Kopfabschnitt zur Lagerung in der Öffnung der Frakturfixierungsplatte aufweisen. Der Kopf kann beispielsweise dazu dienen, ein Durchrutschen der Hülse oder des Gleitstifts durch die Öffnung in der Frakturfixierungsplatte zu verhindern, beispielsweise wenn die Hülse oder der Gleitstift während einer Operation durch die Öffnung der Frakturfixierungsplatte eingeführt wird.

Gemäß einer Ausführungsform ist das gesamte Verankerungselement bei ausschließlichem Zugang von einer knochenabgewandten Seite der Platte einsetzbar, und/oder ist der komplette Schaft des Verankerungselements, jedoch nicht der Kopf des Verankerungselements, durch die Öffnung hindurchführbar. Insbesondere ist das Verankerungselement durch die Öffnung der Platte hindurch in den Knochen einsetzbar. Es kann zuerst die Platte und erst danach das Verankerungselement in den Körper eines Patienten eingebracht werden. Hierdurch kann eine Operation minimal-invasiv durchgeführt werden.

Gemäß einer Ausführungsform sind die Dimensionen des Kopfes und der Öffnung derart aufeinander abgestimmt, dass der Kopf nicht durch die Öffnung hindurchführbar ist. Der Kopf kann eine Auflagerfläche aufweisen, mit der der Kopf im am Knochen fixierten Zustand der Knochenfixierungsvorrichtung am Rand der Öffnung aufliegt. Das kopflose Teil des Schafts kann durch die Öffnung hindurchführbar oder nicht durch die Öffnung hindurchführbar sein.

Insbesondere der Kopf oder Kopfabschnitt und die Öffnung können ferner derart ausgebildet sein, dass vor einer Fixierung die Winkelstellung des Verankerungselements, insbesondere der Hülse oder des Gleitstift, zur Frakturfixierungsplatte innerhalb eines vorgegebenen Winkelbereichs einstellbar ist. Dies ermöglicht eine polyaxiale Einstellung der Lage des Verankerungselements und damit eine gezielte Ausrichtung des Verankerungselements, so dass eine Fraktur zunächst in die richtige Lage gebracht und anschließend die Verankerungselemente derart ausgerichtet werden können, dass möglichst gute Bedingungen für eine Ausheilung der Fraktur erreicht werden können.

Gemäß einer Ausführungsform sind Mittel vorgesehen, um den Kopf des Verankerungselements mit der Platte zu verbinden, insbesondere hinsichtlich der axialen Lage und/oder der Winkelposition des Verankerungselements oder des Teils des Verankerungselements, an dem der Kopf angeordnet ist.

Gemäß einer Ausführungsform weist die Platte im Bereich der Öffnung eine Vertiefung zur teilweisen oder auch vollständigen Aufnahme des Kopfes des Verankerungselements auf. Die Vertiefung ist insbesondere an der knochenabgewandt vorgesehenen Seite der Platte angeordnet.

Der Kopf oder Kopfabschnitt kann ein Außengewinde und die Öffnung oder eine Vertiefung der Öffnung kann ein Innengewinde aufweisen, in welches der Kopfabschnitt eingeschraubt werden kann. Hierdurch kann das Verankerungselement, insbesondere die Hülse oder der Gleitstift, direkt durch Einschrauben in die Frakturfixierungsplatte in dieser verankert werden, wodurch eine besonders stabile Verbindung von Platte und Verankerungselement erreicht wird. Eine derartige Verbindung ist unidirektional, da die Ausrichtung des Verankerungselements vorgegeben ist und damit nur eine Bewegung beispielsweise der Knochenschraube oder des Gleitstifts in der Hülse in Längsrichtung möglich ist.

Es kann ein Blockierelement vorgesehen sein, um den Kopf innerhalb der Öffnung oder einer Vertiefung der Öffnung zu verblocken. Die Öffnung oder die Vertiefung können ein Innengewinde aufweisen, und es kann als Blockierelement ein Verschlusselement, insbesondere eine Verschlussschraube oder ein Verschlussring, mit einem Außengewinde zur Verblockung des Kopfes durch Einschrauben in die Öffnung oder die Vertiefung vorgesehen sein. Die Verschlussschraube dient vor allem dazu, das Verankerungselement in einer bestimmten, insbesondere nach einer Ausrichtung des Verankerungselements ermittelten Winkelstellung fest in der Frakturfixierungsplatte zu verankern, insbesondere um während der Operation ein Verstellen der Winkelstellung des Verankerungselements möglichst zu vermeiden.

Gemäß einer Ausführungsform weist die Platte im zur knochenzugewandten Seite der Platte hin weisenden Bereich der Öffnung oder der Vertiefung der Öffnung eine konkave, das heisst in die Platte hinein gewölbte, Auflagerfläche auf, und/oder der Kopf ist an dem dem Schaft zugewandten Ende ballig ausgeführt, derart, dass vor einer Fixierung das Verankerungselement hinsichtlich seiner Winkellage zur Platte variabel ist.

Die Frakturfixierungsplatte kann zur Versorgung einer Gelenkfraktur, insbesondere einer Fraktur des proximalen Humerus, und/oder zur Anordnung im epiphysären und/oder metaphysären Bereich eines Knochens, insbesondere des proximalen Humerus, ausgebildet sein, insbesondere mehrere Öffnungen zur Lagerung und Fixierung von Verankerungselementen, insbesondere Hülsen oder Gleitstiften, aufweisen, die in einem zur Versorgung des gelenknahen Frakturbereichs vorgesehenen Abschnitt der Frakturfixierungsplatte angeordnet sind. Die Verwendung der Verankerungselemente, insbesondere der gleitend gelagerten stiftförmigen Elemente oder Befestigungselemente wie Knochenschrauben oder Gleitstifte, im gelenksnahen Bereich einer Fraktur kann effizient einer Verletzung der Gelenksfläche entgegen wirken. Bei einer hohen mechanischen Belastung der Gelenksfläche kann sich beispielsweise das stiftförmige Element oder Befestigungselement in der Hülse verschieben, wodurch ein Durchstoßen einer Schrauben- oder Hülsenspitze ins Gelenk weitgehend vermieden werden kann. Weiterhin kann durch die Anordnung von wenigstens zwei Verankerungselementen, insbesondere gleitend gelagerten stiftförmigen Elementen oder Befestigungselementen, im gelenksnahen Bereich eine Rotation von Knochenfragmenten verhindert werden, da die Fraktur bis auf eine Bewegung in Richtung der Achse, insbesondere in Längsrichtung der stiftförmigen Elemente, stabil fixiert werden kann.

Die Knochenfixierungsvorrichtung kann ferner derart ausgebildet sein, dass die Frakturfixierungsplatte im Wesentlichen ohne Kontakt zur Knochenoberfläche fixiert werden kann, insbesondere durch Fixierung des Kopfes des jeweiligen Verankerungselements an der Platte. Dadurch kann das Risiko einer periostalen Durchblutungsstörung an der Knochenoberfläche der Fraktur reduziert werden.

Das stiftförmige Element oder Befestigungselement kann kanüliert sein, insbesondere um einen Führungsdraht zur Positionierung des Elements aufzunehmen. Der Führungsdraht kann beispielsweise zum Vorbohren in den Knochen oder zur Unterstützung beim Ausrichten einer Hülse und des stiftförmigen Elements dienen.

Offenbart ist ferner ein Verankerungselement zur Befestigung einer Frakturfixierungsplatte am Knochen, umfassend einen Schaft sowie einen Kopf, wobei der Schaft wenigstens zweiteilig ausgeführt ist, mit einem ersten Teil, an dem der Kopf angeordnet ist, und mit einem zweiten zur Verankerung im Knochen vorgesehenen Teil, wobei im am Knochen fixierten Zustand der Knochenfixierungsplatte der erste Teil und der zweite Teil entlang einer Achse zueinander verschiebbar geführt sind.

Offenbart ist ferner ein Verfahren zur operativen Versorgung einer Fraktur mit einer Knochenfixierungsvorrichtung, insbesondere wie vorstehend beschrieben, insbesondere ein minimal-invasives Operationsverfahren, bei dem
- eine Frakturfixierungsplatte in den Körper eines Patienten an die Stelle der Fraktur eingebracht wird,
- die Fraktur mit Hilfe der eingebrachten Frakturfixierungsplatte reponiert wird,
- in zumindest einige der Knochenfragmente der Fraktur Bohrungen zur Aufnahme von Verankerungselementen, insbesondere Hülsen oder stiftförmigen Elementen wie Knochenschrauben, eingebracht werden,

- die Verankerungselemente oder Teile hiervon, insbesondere die Hülsen und/oder die stiftförmigen Elementen wie Knochenschrauben oder Gleitstifte, in die Bohrungen eingebracht werden, und bei dem
- die Verankerungselemente winkelstabil in der Frakturfixierungsplatte fixiert werden.

Die Verankerungselemente oder die Teile hiervon können vor oder nach einem endgültigen Einbringen der Frakturfixierungsplatte in den Körper des Patienten in die Bohrungen eingebracht werden.
Beispielsweise kann ein derartiges Operationsverfahren zur schonenden Versorgung einer proximalen Humerusfraktur eingesetzt werden.

Weitere Ausgestaltungen der Erfindung sind Gegenstand der abhängigen Ansprüche.

Selbstverständlich können die Merkmale der oben angegebenen Ausführungsformen der Erfindung jeweils untereinander zu spezifischen Ausführungsformen kombiniert werden.

### Kurze Beschreibung der Zeichnung

Die Erfindung wird nachfolgend anhand von in der Zeichnung dargestellten Ausführungsbeispielen näher erläutert. Im Einzelnen zeigen
- Fig. 1: als ein Ausführungsbeispiel einer Knochenfixierungsvorrichtung eine Humerusplatte, bei der drei Verankerungselemente im proximalen Abschnitt der Platte angeordnet sind;
- Fig. 2: als ein weiteres Ausführungsbeispiel einer Knochenfixierungsvorrichtung eine Humerusplatte, teilweise im Schnitt, mit einem polyaxial ausrichtbaren Verankerungselement;
- Fig. 3: das in die Humerusplatte von Fig. 2 eingesetzte Verankerungselement im Längsschnitt;
- Fig. 4: als ein weiteres Ausführungsbeispiel einer Knochenfixierungsvorrichtung eine Humerusplatte mit drei unidirektional ausrichtbaren Verankerungselementen;
- Fig. 5: eines der in die Humerusplatte von Fig. 4 eingesetzten Verankerungselemente im Längsschnitt;
- Fig. 6: eine Konstruktionszeichnung eines polyaxial ausrichtbaren Verankerungselements für eine Humerusplatte;
- Fig. 7: eine Konstruktionszeichnung eines unidirektional ausrichtbaren Verankerungselements für eine Humerusplatte;
- Fig. 8: das in Fig. 7 gezeigte Verankerungselement in perspektivischer Darstellung;
- Fig. 9: ein Ausführungsbeispiel eines Verankerungselements, bei dem an der Spitze eines stiftförmigen Elements ein kegelförmiger oder kegelstumpfförmiger Verankerungsabschnitt angeordnet ist;
- Fig. 10: ein Ausführungsbeispiel eines Verankerungselements, bei dem an der Spitze eines stiftförmigen Elements ein kurzer Gewindeabschnitt zum Verankern im Knochen angeordnet ist;
- Fig. 11: ein Ausführungsbeispiel eines Verankerungselements, bei dem ein stiftförmiges Element in Form eines Gleitstifts gleitend in einer Hülse gelagert ist, die mit ihrem Kopfabschnitt in einer Humerusplatte winkelstabil fixiert werden kann;
- Fig. 12: ein Ausführungsbeispiel eines Verankerungselements mit einer im Knochen verankerten Hülse mit Außengewinde, in der ein Gleitstift, der winkelstabil mit seinem Kopfabschnitt in einer Humerusplatte verankert werden kann, gleitend gelagert ist;
- Fig. 13: ein Ausführungsbeispiel eines polyaxial ausrichtbaren Verankerungselements mit Verschlussschraube;
- Fig. 14: ein weiteres Ausführungsbeispiel eines polyaxial ausrichtbaren Verankerungselements mit Verschlussschraube; und
- Fig. 15: ein Ausführungsbeispiel eines unidirektional ausrichtbaren Verankerungselements mit einem Kopf mit Außengewinde.

Die nachfolgende Beschreibung der Ausführungsbeispiele und die Zeichnungen dienen dem besseren Verständnis der Erfindung und sollen nicht zur Einschränkung der in den Ansprüchen gekennzeichneten Erfindung herangezogen werden.

### Wege zur Ausführung der Erfindung

Ausführungsbeispiele der Erfindung werden im Folgenden am Beispiel einer Knochenfixierungsvorrichtung zur operativen Versorgung einer Humerusfraktur, insbesondere einer proximalen Humerusfraktur beschreiben, obwohl die Erfindung nicht auf eine derartige Anwendung beschränkt ist, sondern für jede Art von Frakturversorgung verwendet werden kann. Insbesondere kann die Knochenfixierungsvorrichtung auch an anderen Knochen als am Humerus, insbesondere auch an der Tibia oder dem Femur, zur Frakturfixierung verwendet werden. Besonders im gelenksnahen Bereich zeigt jedoch die Erfindung insbesondere aufgrund der reduzierten Kompressionswirkung deutliche Vorteile gegenüber herkömmlichen Fixierungsvorrichtungen hinsichtlich der Belastbarkeit von Gelenken während des Heilungsprozesses und der Verringerung des Risikos von Gelenkverletzungen insbesondere durch Belastung und dadurch erhöhter Kompression.

Im Folgenden wird der Begriff "Gleitschraube" als Bezeichnung für die Kombination aus einer Hülse und einer darin entlang ihrer Längsachse gleitend geführten Knochenschraube oder eines darin entlang ihrer Längsachse gleitend geführten Gleitstifts im Sinne eines stiftförmigen Elements oder Befestigungselements verwendet. Die Gleitschraube dient hier im Sinne der Erfindung als ein Verankerungselement oder eine Gleitverankerung der Humerusplatte am Knochen.

Die im Folgenden als Beispiel für Frakturfixierungsplatten beschriebenen Humerusplatten, die im fixierten Zustand im Wesentlichen keinen Kontakt zur Knochenoberfläche haben, basieren auf dem Prinzip der winkelstabilen Plattenfixierung, bei der in der Humerusplatte gelagerte Gleitschrauben winkelstabil verankert werden. Hierzu stehen zwei unterschiedliche Arten der Fixierung der Gleitschrauben zur Verfügung:
1. unidirektionale oder uniaxiale Fixierung: die Winkelstellung einer Gleitschraube ist nicht frei wählbar, da sie durch die Öffnung in der Platte, durch welche die Hülse der Gleitschraube oder das stiftförmige Element geführt ist, und die Fixierung der Gleitschraube in der Platte vorgegeben ist.
2. polyaxiale oder polydirektionale Fixierung: die Gleitschraube kann in der Öffnung der Platte unter variierenden Winkelstellungen fixiert werden, typischerweise in Winkeln bis ca. 30°. Die Fixierung ist also nicht von vornherein festgelegt, sondern kann beispielsweise durch Klemmung der Hülse der Gleitschraube oder des stiftförmigen Elements in der Öffnung erfolgen. Dadurch kann eine Anpassung der Lage der Gleitschraube an die Anatomie des Knochens erreicht werden.

In beiden Fixierungsfällen ist die Humerusplatte an eine durchschnittliche Anatomie des Humerus angepasst. Allgemeiner ist die Form der jeweiligen Knochenfixierungsplatte an die Form des jeweils zu fixierenden Knochens oder Knochentyps angepasst.

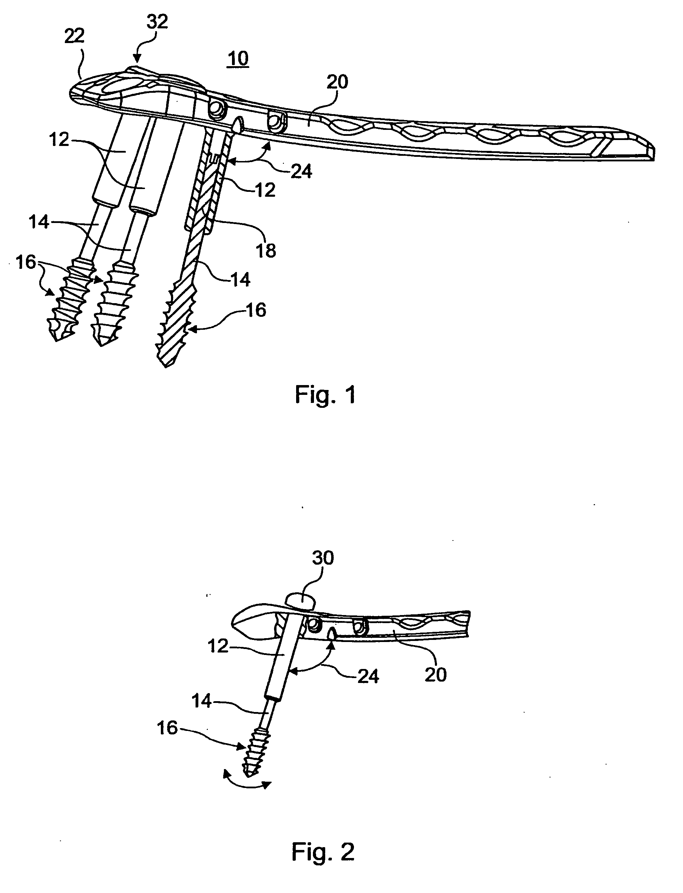

Die in Fig. 1 gezeigte Knochenfixierungsvorrichtung 10 umfasst eine Humerusplatte 20. Die Humerusplatte 20 mit einer ersten, in Fig. 1 nach oben gerichteten, einem Knochen (nicht gezeigt) abgewandten Seite und mit einer zweiten, in Fig. 1 nach unten gerichteten, dem Knochen zugewandten Seite ist mit drei eingeführten und an der Platte 20 fixierten Verankerungselementen oder Gleitschrauben versehen. Die knochenabgewandte Seite der Platte 20 ist diejenige Seite der Platte 20, auf der sich im am Knochen fixierten Zustand der Knochenfixierungsvorrichtung der Kopf einer Schraube befindet. Die Gleitschrauben umfassen jeweils eine Hülse 12 und eine darin gleitend geführte Knochenschraube 14. Die Hülse 12 und die Knochenschraube 14 bilden einen zweiteiligen Schaft der Gleitschraube. Die Hülse 12 und die Knochenschraube 14 sind auch im am Knochen fixierten Zustand der Knochenfixierungsvorrichtung, d.h. insbesondere im bestimmungsgemäßen Gebrauch oder Zustand der Knochenfixierungsvorrichtung, entlang ihrer gemeinsamen Längsachse zueinander frei verschiebbar geführt. Die Gleitschraube ist daher teleskopisch ausziehbar. Quer zu der Achse ist die relative Lage der beiden Teile zueinander unveränderlich oder fixiert.

Die Hülsen 12 sind in der Humerusplatte 20 gelagert. Hierzu sind die Hülsen 12 durch Öffnungen 22 in der Platte 20 geführt, die sich von der ersten Seite der Platte 20 durch die Platte 20 hindurch zu der zweiten Seite der Platte 20 erstrecken, so dass sie jeweils in eine in den Knochen eingebrachte Bohrung (nicht gezeigt) hineinragen. Jede der Hülsen 12 kann in einem bestimmten Winkel 24 zur Humerusplatte 20 fixiert und damit optimal an die Anatomie des Knochens und der zu fixierenden Fraktur angepasst werden. Die Humerusplatte 10 weist einen gelenksnahen Abschnitt 32 auf, der zur Versorgung des gelenknahen Frakturbereichs des Humerus ausgebildet, d.h. entsprechend anatomisch geformt ist. In diesem Abschnitt 32 sind die drei Gleitschrauben angeordnet, die somit wirksam eine Rotation von Knochenfragmenten verhindern können. Bis auf eine Bewegung in Längsrichtung der Gleitschraube kann somit eine Fraktur stabilisiert werden.

Jede Knochenschraube 14 weist einen Gewindeabschnitt 16 als Verankerungsabschnitt auf, der in den Knochen zur Fixierung der Fraktur hineingedreht wird. Ferner besitzt jede Knochenschraube 14 einen Gleitabschnitt 18 mit einem Außenführungsbereich in Form eines gewindelosen Schraubenabschnitts, der sich an den Gewindeabschnitt 16 anschließt und sich zumindest teilweise in einer in der Humerusplatte 20 fixierten Hülse 12 mit einem Innenführungsbereich befindet. In der Hülse 12 kann sich der Gleitabschnitt 18 lineargeführt in Richtung der Längsachse der im Knochen verankerten Knochenschraube 14 frei gleitend bewegen, wodurch im Wesentlichen keine auf die Knochenschraube 14 wirkenden Längskräfte, die beispielsweise bei einer Belastung der Knochens auftreten, auf die Knochenfixierungsvorrichtung übertragen werden. Der Außenführungsbereich des Gleitabschnitts 18 und der Innenführungsbereich der Hülse 12 weisen jeweils einen in Richtung ihrer Längsachse konstanten Querschnitt auf, d.h. sie sind jeweils zylindrisch ausgeführt. Quer zu der Längsachse gewährleisten der Außenführungsbereich und der Innenführungsbereich eine Führung von Hülse 12 und stiftförmigen Element 14.

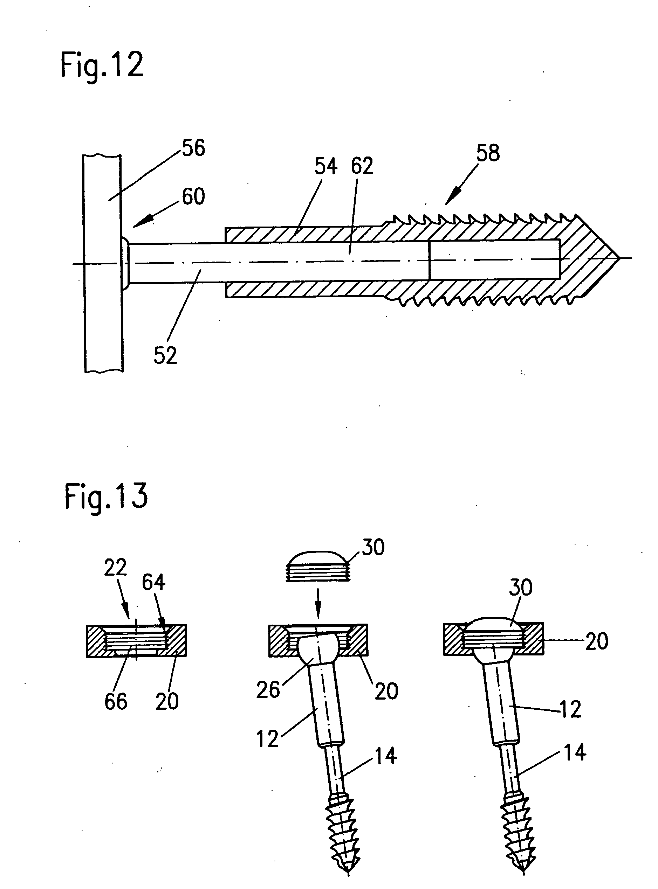

Fig. 2 zeigt eine polyaxial in der Humerusplatte 20 fixierbare Gleitschraube umfassend eine Hülse 12 und Knochenschraube 14. Durch die polyaxiale Fixierung kann der Winkel 24 zwischen der Gleitschraube und der Humerusplatte 20 bis ca. 30° variiert werden. Dies ermöglicht eine gezielte Ausrichtung jeder einzelnen Hülse 12 und Knochenschraube 14, insbesondere an die Anatomie und Fraktur des Knochens. Die Fixierung der Hülse 12 in der Humerusplatte 20 erfolgt kraftschlüssig durch eine Verschlussschraube 30, die in die Öffnung 22 der Humerusplatte 20 eingeschraubt wird und die Hülse 12 in der beabsichtigten Winkelstellung festklemmt, wie in Fig. 13 gezeigt ist, so dass eine nachträgliche Veränderung der Winkelstellung verhindert wird. Dadurch kann ein Chirurg eine Fraktur in die korrekte Lage bringen und fixiert halten, wodurch optimale Bedingungen für die Ausheilung der Fraktur erreicht werden.

In Fig. 13 ist eine Humerusplatte 20 mit einer polyaxialen Gleitschraube im Bereich einer Öffnung 22 dargestellt, wobei die Platte 20 in drei verschiedenen Konfiguration gezeigt ist, nämlich alleine, mit eingesetzter Gleitschraube und mit fixierter Gleitschraube. Die Öffnung 22 der Humerusplatte 20 weist auf der knochenabgewandten Seite der Humerusplatte 20 eine Vertiefung 66 auf, in der ein Kopf 26 der Gleitschraube vollständig aufgenommen ist. Der Kopf 26 ist an einem manipulationsseitigen Ende der Gleitschraube, in der Ausführungsform gemäß Fig. 13 an einem manipulationsseitigen Ende der Hülse 12, angeordnet. Der Kopf 26 schließt an den Schaft der Gleitschraube an. Die Platte weist an einem zu der knochenzugewandten Seite hin weisenden Bereich der Öffnung 22 oder der Vertiefung 66 eine konkave Auflagerfläche auf. Der Kopf 26 ist zumindest an dem dem Schaft zugewandten Ende ballig ausgeführt, um eine Gegenform zu der konkaven Auflagerfläche der Öffnung 22 zu bilden. Die Winkellage der Gleitschraube bezüglich des Humerusplatte 20 ist daher grundsätzlich variabel. Die Gleitschraube ist, bis auf den Kopf 26, vollständig durch die Öffnung 22 hindurchführbar.

Darüber hinaus weist die Öffnung 22 auf der knochenabgewandten Seite der Humerusplatte 20, insbesondere die Vertiefung 66, ein Innengewinde 64 auf. In das Innengewinde 64 ist die Verschlussschraube 30, welche ein dem Innengewinde 64 entsprechendes Außengewinde aufweist, einschraubbar, um in der jeweils gewählten Winkelstellung der Gleitschraube den Kopf 26 der Gleitschraube gegen die Auflagerfläche der Vertiefung 66 zu drücken. Hierdurch wird der Kopf 26 innerhalb der Öffnung 22 derart verblockt, dass die Winkellage der Gleitschraube zu der Humerusplatte 20 fixiert ist. Hierdurch wird der Kopf 26 der Gleitschraube mit der Humerusplatte 20 verbunden, insbesondere fest oder starr verbunden, beispielsweise durch Kraftschluss oder Formschluss.

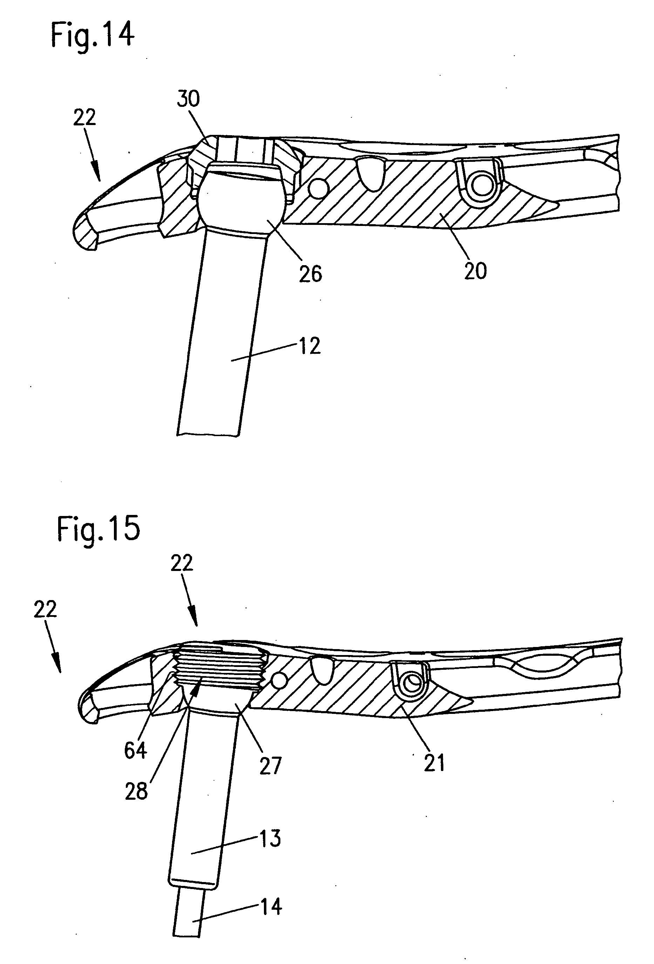

Eine weitere Ausführungsform einer Knochenfixierungsvorrichtung, die im Wesentlichen der in Fig. 13 gezeigten Ausführungsform entspricht, ist in Fig. 14 dargestellt, wobei der Einfachheit halber auf die Darstellung von Gewinden verzichtet wurde.

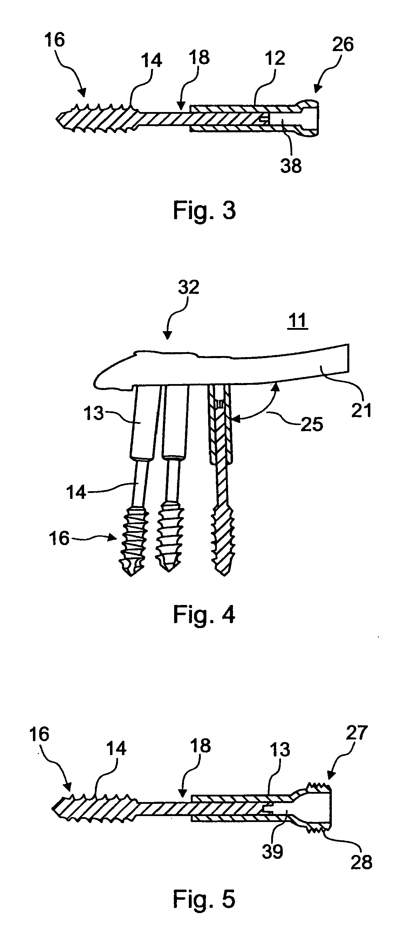

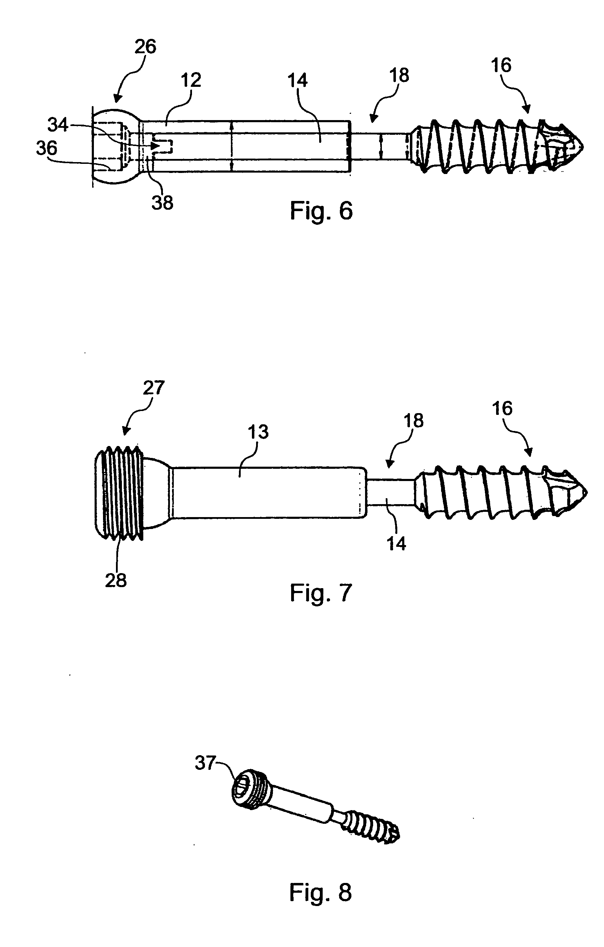

Die verwendete Hülse 12 und die darin gleitend geführte Knochenschraube 14 sind in Fig. 3 und in Fig. 6 anhand einer Konstruktionszeichnung im Längsschnitt gezeigt. Der Kopfabschnitt 26 der Hülse 12 ist, wie vorstehend bereits erläutert, ähnlich wie eine Halbkugel ausgebildet zur Lagerung in der komplementär ausgebildeten Öffnung 22 in der Humerusplatte 20. Aufgrund der Form des Kopfabschnitts 26 und der komplementären Ausbildung der Öffnung 22 in der Humerusplatte 20 kann die Hülse 12 vor der Fixierung vom Chirurg bequem durch entsprechendes Verschwenken der Gleitschraube ausgerichtet werden. Zur Erleichterung der Ausrichtung kann die Knochenschraube 14 kanüliert sein, d.h. einen durchgängigen Längskanal besitzen, durch den ein Führungsdraht geführt werden kann, mit dem die Gleitschraube in die gewünschte Position gebracht werden kann.

Einen Anschlag für die Knochenschraube 14 bildet deren Gewindeabschnitt 16, wodurch ein zu tiefes Eingleiten in die Hülse 12 verhindert wird. Zudem ist die Länge des Gleitabschnitts 18 derart bemessen, dass er beim Anschlag des Gewindeabschnitts 16 an der Hülse 12 nicht auf der anderen Seite der Hülse 12 austritt.

Um das Eindrehen der kopflosen Knochenschrauben 14 in den Knochen zu ermöglichen, ist einerseits ein Durchgangskanal 38 durch den Kopfabschnitt 26 und für ein Werkzeug (nicht gezeigt) zum Eindrehen der Knochenschraube 14 vorgesehen und andererseits das Ende des gewindelosen Gleitabschnitts 18 jeder Knochenschraube 14 mit einem Schlitz 34 versehen (siehe Fig. 6). Es ist auch möglich, anstelle eines Schlitzes andere Vorrichtungen zum Eindrehen der Knochenschraube 14 vorzusehen, beispielsweise einen Innensechskant. Nach Ausrichten der Hülse 12 und Knochenschraube 14 kann durch den Durchgangskanal 38 ein Werkzeug wie ein Schraubendreher geführt werden, dessen Spitze in den Schlitz 34 eingreift und ein Eindrehen des Gewindeabschnitts 16 der Knochenschraube 14 in den Knochen ermöglicht. Im Kopfabschnitt 26 der Hülse 12 ist ferner ein Innensechskant 36 vorgesehen, in den ein entsprechendes Werkzeug (nicht gezeigt) eingreifen kann, welches das Ausrichten der Hülse 12 und auch zumindest teilweise Einbringen der Hülse 12 in eine Bohrung im Knochen erleichtert.

Fig. 4 zeigt eine Knochenfixierungsvorrichtung 11 mit unidirektional in einer Humerusplatte 21 fixierten Hülsen 13. Jeder Kopfabschnitt 27 einer Hülse 13 weist ein Außengewinde 28 auf (Fig. 5, 7, 8), das in ein entsprechendes Innengewinde (in Fig. 4 nicht gezeigt) einer Öffnung 22 der Humerusplatte 21 oder einer Vertiefung 66 der Öffnung 22 zur Aufnahme und Lagerung der Hülse 13 geschraubt werden kann. Dadurch weist jede Knochenschraube 14 und Hülse 13 (Gleitschraube) einen vorgegebenen Winkel 25 zur Humerusplatte 21 auf. Eine Variation des Winkels 25 ist nicht vorgesehen. Fig. 5 zeigt die Hülse 13 und die darin gleitend geführte Knochenschraube 14 im Längsschnitt. Fig. 7 zeigt die Kombination aus Hülse 13 und Schraube 14 in einer Konstruktionszeichnung in Seitenansicht, und Fig. 8 zeigt die Kombination in einer perspektivischen Ansicht. Insbesondere in Fig. 8 ist zu erkennen, dass der Kopfabschnitt 27 der Hülse einen Innensechskant 37 aufweist, in den ein entsprechendes Werkzeug (nicht gezeigt) zum Einschrauben des Kopfabschnitts 27 in eine Öffnung 22 in der Humerusplatte 21 eingreifen kann.

Fig. 15 zeigt eine Ausführungsform einer Knochenfixierungsvorrichtung, die im Wesentlichen der in Fig. 4 gezeigten Ausführungsform entspricht, wobei ein Außengewinde 28 eines Kopfes 27 einer Hülse 13 in ein Innengewinde 64 einer Öffnung 22 einer Knochenfixierungsplatte 21 eingreift, um den Kopf 27 an der Platte 21 unidirektional zu fixieren.

Grundsätzlich ist es sowohl bei einer polyaxialen als auch unidirektionalen Fixierung jedoch auch möglich, dass keine Vertiefung vorgesehen ist, und/oder eine Öffnung ein Innengewinde für eine Verschlussschraube oder einen Gleitschraubenkopf aufweist. Insbesondere kann eine derartige Öffnung auch ein durchgehendes Innengewinde aufweisen.

Es kann grundsätzlich auch vorgesehen sein, dass zur Durchführung einer Gleitschraube durch eine ein Innengewinde aufweisende Öffnung in einer Frakturfixierungsplatte ein Gewindeabschnitt der Gleitschraube, der zur Verankerung der Gleitschraube im Knochen ausgebildet ist, durch die Öffnung hindurch geschraubt werden muss. In diesem Fall ist der Außendurchmesser des Gewindeabschnitts der Gleitschraube größer als der kleinste Innendurchmesser der Öffnung der Frakturfixierungsplatte und der Kerndurchmesser des Gewindeabschnitts der Gleitschraube ist kleiner als der kleinste Innendurchmesser der Öffnung.

Im Folgenden wird beschrieben, wie die in Fig. 1 gezeigte Knochenfixierungsvorrichtung operativ implantiert werden kann. Die Humerusplatte 20 kann offen, d.h. mittels Darstellung der Fraktur, oder minimal-invasive unter Verwendung eines speziellen (nicht dargestellten) Zielgeräts in den Körper eingebracht werden.

Nach Einbringen der Platte 20 wird die Fraktur des Humerus reponiert unter Benutzung der eingebrachten Platte 20 als Schablone für ein Zielgerät. Der Zielbügel des Zielgeräts ist als zweiteiliges Instrument ausgebildet. Die Reposition der Fraktur wird anschließend per Röntgenbild kontrolliert.

Um die Platte 20 in einem vorgegebenen Abstand zur Knochenoberfläche winkelstabil fixieren zu können, können Abstandshalter durch die Öffnungen 22 der Humerusplatte 20 zwischen Platte 20 und Knochenoberfläche eingebracht werden. Die Abstandshalter werden nach der endgültigen Positionierung und Fixierung der Platte 20 wieder entfernt.

Nun erfolgt ein Vorbohren in den Knochen. Entsprechend der Länge der Hülsen 12 wird der Außendurchmesser aufgebohrt. Darauf folgend wird der Kerndurchmesser der Gleitschraube, d.h. der Knochenschraube 14 aufgebohrt. Dieser Vorgang kann zur besseren Kontrolle unter Röntgenbeleuchtung durchgeführt werden.

Anschließend werden die Gleitschrauben durch die entsprechenden Öffnungen 22 der Humerusplatte 20 in die Bohrungen im Knochen eingesetzt und die Knochenschrauben 14 in den Knochen eingedreht. Bei der polyaxialen Platte 20 werden nun die Winkelstellungen der Gleitschrauben mit Klemmschrauben 30 fixiert. Bei der unidirektionalen Platte 21 erfolgt die Fixierung der Hülsen 13 durch Einschrauben der Hülsen 13 in die Platte 21.

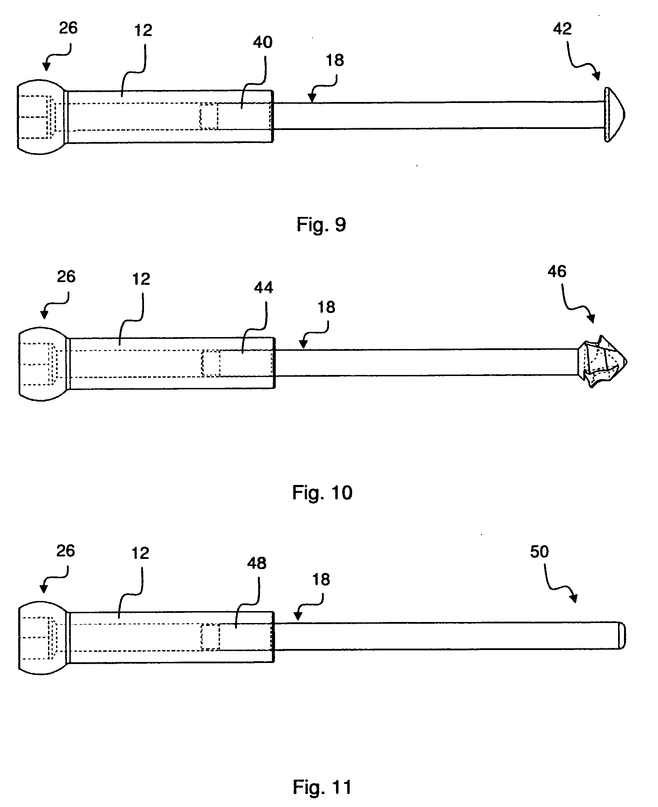

Fig. 9 zeigt eine weitere Ausführungsform einer polyaxial in einer Humerusplatte 20 fixierbaren Gleitverankerung, umfassend eine Hülse 12 für die polyaxiale Fixierung der Gleitverankerung in der Humerusplatte 20 und ein darin gleitend gelagertes stiftförmiges Element oder Befestigungselement in Form eines Gleitstifts 40, der an seiner Spitze zur Verankerung im Knochen einen kegelförmigen oder kegelstumpfförmigen Verankerungsabschnitt 42 aufweist, der kein Gewinde aufweist.

Eine ähnliche Ausführungsform einer Gleitverankerung ist in Fig. 10 gezeigt. Bei dieser Ausführungsform besitzt ein Gleitstift 44 an seiner Spitze ebenfalls einen kegelförmigen oder kegelstumpfförmigen Verankerungsabschnitt 46, der allerdings im Unterschied zum in Fig. 9 gezeigten Ausführungsbeispiel ein Gewinde besitzt (sogenannte Gewindekugel), was das Einbringen des Gleitstifts 44 in die Spongiosa etwas erleichtern kann. Das Gewinde weist in der gezeigten Ausführungsform nur einen Gewindegang und sogar nur einen halben Gewindegang auf und ist daher mit wenigen Umdrehungen im Knochen verankerbar.

In Fig. 11 ist weiterhin ein Ausführungsbeispiel einer Gleitverankerung gezeigt, die sich von den in den Fig. 9 und 10 gezeigten Ausführungsbeispielen darin unterscheidet, dass ein Gleitstift 48 einen gewindelosen Verankerungsabschnitt 50 aufweist und daher als eine Art Bolzen implementiert ist. Im Prinzip geht bei diesem Gleitstift 48 der Gleitabschnitt18 unmittelbar in den gewindelosen Verankerungsabschnitt 50 über. Dieser Gleitstift 48 kann daher relativ einfach in eine Bohrung im Knochen eingebracht werden, insbesondere durch Einstecken in ein vorgebohrtes Loch im Knochen.

Schließlich zeigt Fig. 12 ein Ausführungsbeispiel einer im Schnitt dargestellten Gleitverankerung, die einen Gleitstift 52 mit Gleitabschnitt 62 umfasst, der an einem Ende in einer Humerusplatte 56 und am anderen Ende in einer Hülse 54 gelagert ist, die ein Außengewinde 58 zum Einschrauben in einen Knochen aufweist. Der Gleitstift 52 kann polyaxial in der Humerusplatte 56 fixiert werden, beispielsweise nach Art der Fig. 13 oder Fig. 14, und ist gleitend in der Hülse 54 gelagert, die wiederum in den Knochen eingeschraubt und dadurch darin verankert ist. Der Vorteil einer derartigen Ausführung der Gleitverankerung ist die große erzielbare Polyaxialität, da der Durchmesser des Gleitstifts 52 wesentlich kleiner ausgebildet sein kann als die Öffnung 22 in der Humerusplatte 56, in welcher ein nur abschnittsweise erkennbarer Kopf 60 des Gleitstifts 52 gelagert ist, wobei der Kopf 60 an dem dem Gleitstift 52 zugewandten Ende ballig ausgeführt ist. Dies ermöglicht auch die Einstellung sehr kleiner Winkel zwischen Humerusplatte 56 und dem Gleitstift 52. Der Gleitstift kann aber auch unidirektional in der Humerusplatte 56 fixiert werden, beispielsweise nach Art der Fig. 15.

Weiterhin kann bei einer operativen Implantierung zunächst die Hülse 54 an einer geeigneten Stelle in einen Knochen oder ein Knochenfragment eingebracht werden. Die Hülse 54 muss in diesem Fall nicht durch die Öffnung 22 in der Humerusplatte 56 hindurchführbar sein. Durch die Hülse 54, die relativ zu dem Gleitstift 52 einen vergleichsweise großen Querschnitt besitzt, kann eine gute Verankerung auch in einem Knochen mit geringerer Qualität gewährleistet werden. Danach kann die Humerusplatte 56, gegebenenfalls mit darin gelagertem Gleitstift 52, außen am Knochen angebracht und durch zumindest teilweises Einführen des Gleitstifts 52 in die im Knochen verankerte Hülse 54 fixiert werden. Der Gleitstift 52 kann ferner winkelstabil in der Humerusplatte 56 fixiert werden, um ein optimales Reponieren einer Fraktur bei gleichzeitiger Kompressionswirkung zu erzielen. Selbstverständlich kann auch vorgesehen sein, das die Hülse und die Öffnung so dimensioniert sind, dass die Hülse durch die Öffnung hindurchführbar ist, und also wiederum der gesamte Schaft des Verankerungselements durch die Öffnung hindurchführbar ist.

Im Lichte der hier gemachten Ausführungen eröffnen sich dem Fachmann weitere Ausführungsformen der in den Ansprüchen gekennzeichneten Erfindung, welche hier nicht abschließend dargestellt werden können.

### Bezugszeichenliste

- 10: polyaxiale Knochenfixierungsvorrichtung
- 11: unidirektionale Knochenfixierungsvorrichtung
- 12: Hülse für polyaxiale Fixierung in der Humerusplatte 20
- 13: Hülse für unidirektionale Fixierung in der Humerusplatte 21
- 14: Knochenschraube
- 16: Gewindeabschnitt
- 18: Gleitabschnitt
- 20: Humerusplatte mit polyaxialer Fixierung von Gleitschrauben
- 21: Humerusplatte mit unidirektionaler Fixierung von Gleitschrauben
- 22: Öffnung zur Lagerung und Fixierung der Hülse
- 24: Winkel zwischen Hülse 12 und Humerusplatte 20
- 25: Winkel zwischen Hülse 13 und Humerusplatte 21
- 26: Kopfabschnitt der Hülse 12
- 27: Kopfabschnitt der Hülse 13
- 28: Außengewinde des Kopfabschnitts 27
- 30: Verschlussschraube zur Fixierung der Hülse 12
- 32: zur Versorgung des gelenknahen Frakturbereichs vorgesehener Abschnitt der Humerusplatte 20 bzw. 21
- 34: Schlitz zum Eindrehen der Knochenschraube 14
- 36: Innensechskant des Kopfabschnitts 26 der Hülse 12
- 37: Innensechskant des Kopfabschnitts 27 der Hülse 13
- 38: Durchgangskanal durch den Kopfabschnitt 26 der Hülse 12
- 39: Durchgangskanal durch den Kopfabschnitt 27 der Hülse 13
- 40: stiftförmiges Element
- 42: kegel(stumpf)förmiger Verankerungsabschnitt
- 44: stiftförmiges Element
- 46: kegel(stumpf)förmiger Verankerungsabschnitt mit Gewinde
- 48: stiftförmiges Element
- 50: gewindeloser Verankerungsabschnitt
- 52: Gleitstift
- 54: Hülse
- 56: Humerusplatte
- 58: Außengewinde der Hülse 54
- 60: Kopf des Gleitstifts 52
- 62: Gleitabschnitt des Gleitstifts 52
- 64: Innengewinde der Öffnung 22
- 66: Vertiefung der Öffnung 22

## Patentansprüche

1. Knochenfixierungsvorrichtung (10; 11) zur operativen Versorgung von Frakturen, umfassend
eine Frakturfixierungsplatte (20; 21; 56), die wenigstens eine Öffnung (22) aufweist, und
wenigstens ein Verankerungselement zur Befestigung der Frakturfixierungsplatte am Knochen, wobei das Verankerungselement einen Schaft sowie einen Kopf (26, 27; 60) aufweist, und wobei wenigstens ein Teil des Schafts durch die Öffnung hindurchführbar ist, während der Kopf in der Öffnung fixierbar ist,
und wobei der Schaft wenigstens zweiteilig ausgeführt ist, mit einem ersten Teil (12, 13, 40, 44, 48; 52), an dem der Kopf angeordnet ist, und mit einem zweiten zur Verankerung im Knochen vorgesehenen Teil (14; 54), **dadurch gekennzeichnet, dass** im am Knochen fixierten Zustand der Knochenfixierungsvorrichtung der erste Teil und der zweite Teil entlang einer Achse zueinander verschiebbar geführt sind.

2. Knochenfixierungsvorrichtung gemäß Anspruch 1, **dadurch gekennzeichnet, dass** das Verankerungselement eine Hülse (12, 13; 54) mit einem Innenführungsbereich und ein darin aufgenommenes, insbesondere stiftförmiges Element (14, 40, 44, 48; 52) mit einem Außenführungsbereich umfasst, welche Bereiche in Richtung der Achse jeweils einen konstanten Querschnitt, insbesondere einen kreisförmigen Querschnitt, aufweisen, derart, dass der Außenführungsbereich im Innenführungsbereich lineargeführt in Richtung der Achse gleiten kann.

3. Knochenfixierungsvorrichtung gemäß einem der vorstehenden Ansprüche, **dadurch gekennzeichnet, dass** vor einer Fixierung die Winkelstellung (24) des Verankerungselements zur Platte innerhalb eines vorgegebenen Bereichs frei einstellbar ist.

4. Knochenfixierungsvorrichtung gemäß einem der vorstehenden Ansprüche, **dadurch gekennzeichnet, dass** Mittel (22, 28, 30) vorgesehen sind, um den Kopf des Verankerungselements mit der Platte zu verbinden.

5. Knochenfixierungsvorrichtung gemäß einem der vorstehenden Ansprüche, **dadurch gekennzeichnet, dass** die Platte im Bereich der Öffnung eine Vertiefung (66) zur teilweisen oder auch vollständigen Aufnahme des Kopfes des Verankerungselements aufweist.

6. Knochenfixierungsvorrichtung gemäß einem der vorstehenden Ansprüche, **dadurch gekennzeichnet, dass** der Kopf ein Außengewinde (28) aufweist und die Öffnung oder eine Vertiefung (66) der Öffnung ein Innengewinde (22) aufweist, in welches der Kopf einschraubbar ist.

7. Knochenfixierungsvorrichtung gemäß einem der vorstehenden Ansprüche, **dadurch gekennzeichnet, dass** ein Blockierelement (30) vorgesehen ist, um den Kopf innerhalb der Öffnung oder einer Vertiefung (66) der Öffnung zu verblocken.

8. Knochenfixierungsvorrichtung gemäß Anspruch 7, **dadurch gekennzeichnet, dass** die Öffnung oder die Vertiefung ein Innengewinde (22) aufweist, und als Blockierelement ein Verschlusselement, insbesondere eine Verschlussschraube (30) oder ein Verschlussring, mit einem Außengewinde zur Verblockung des Kopfes durch Einschrauben in die Öffnung oder die Vertiefung vorgesehen ist.

9. Knochenfixierungsvorrichtung gemäß einem der vorstehenden Ansprüche, **dadurch gekennzeichnet, dass** die Platte im zur knochenzugewandten Seite der Platte hin weisenden Bereich der Öffnung oder der Vertiefung der Öffnung eine konkave Auflagerfläche aufweist und/oder der Kopf an dem dem Schaft zugewandten Ende ballig ausgeführt ist, derart, dass vor einer Fixierung das Verankerungselement hinsichtlich seiner Winkellage zur Platte variabel ist.

10. Knochenfixierungsvorrichtung (10; 11) gemäß einem der vorhergehenden Ansprüche, **dadurch gekennzeichnet, dass** sie derart ausgebildet ist, dass die Frakturfixierungsplatte (20; 21) im Wesentlichen ohne Kontakt zur Knochenoberfläche fixierbar ist.

11. Knochenfixierungsvorrichtung (10; 11) gemäß einem der vorhergehenden Ansprüche, **dadurch gekennzeichnet, dass** die Frakturfixierungsplatte (20; 21) zur Anordnung im epiphysären und/oder metaphysären Bereich eines Knochens, insbesondere des proximalen Humerus, ausgebildet ist.

12. Knochenfixierungsvorrichtung gemäß einem der vorstehenden Ansprüche, **dadurch gekennzeichnet, dass** die Dimensionen des Kopfes und der Öffnung derart aufeinander abgestimmt sind, dass der Kopf nicht durch die Öffnung hindurchführbar ist.

13. Knochenfixierungsvorrichtung gemäß einem der vorstehenden Ansprüche, **dadurch gekennzeichnet, dass** der komplette Schaft des Verankerungselements, jedoch nicht der Kopf des Verankerungsetements, durch die Öffnung hindurchführbar ist.

14. Knochenfixierungsvorrichtung gemäß einem der Ansprüche 2 bis 13, **dadurch gekennzeichnet, dass** das insbesondere stiftförmige Element zur Verankerung im Knochen und die Hülse zur Fixierung in der wenigstens einen Öffnung der Frakturfixierungsplatte ausgebildet ist.

15. Knochenfixierungsvorrichtung gemäß einem der Ansprüche 2 bis 13, **dadurch gekennzeichnet, dass** die Hülse zur Verankerung im Knochen und das insbesondere stiftförmige Element zur Fixierung in der wenigstens einen Öffnung der Frakturfixierungsplatte ausgebildet ist.

## Claims

1. A bone fixing apparatus (10; 11) for the surgical care of fractures, comprising
a fracture fixing plate (20; 21; 56) which has at least one opening (22); and
at least one anchorage element for the fastening of the fracture fixing plate to the bone, wherein the anchorage element has a shaft as well as a head (26, 27; 60) and wherein at least a part of the shaft is able to be passed through the opening, whereas the head can be fixed in the opening,
and wherein the shaft is designed in at least two parts, with a first part (12, 13, 40, 44, 48; 52) at which the head is arranged and with a second part (14; 54) provided for the anchorage in the bone,
**characterized in that**, in the state in which the bone fixing apparatus is fixed to the bone, the first part and the second part are guided displaceably with respect to one another along an axis.

2. A bone fixing apparatus in accordance with claim 1, **characterized in that** the anchorage element comprises a sleeve (12, 13; 54) with an inner guide region and an element (14, 40, 44, 48; 52), in particular of pin-shape, received therein and having an outer guide region, said regions each having a constant cross-section in the direction of the axis, in particular a circular cross-section, such that the outer guide region can slide in a linearly guided manner in the direction of the axis in the inner guide region.

3. A bone fixing apparatus in accordance with any one of the preceding claims **characterized in that** the angular position (24) of the anchorage element with respect to the plate can be freely set within a predefined range before the fixing.

4. A bone fixing apparatus in accordance with any one of the preceding claims, **characterized in that** means (22, 28, 30) are provided to connect the head of the anchorage element to the plate.

5. A bone fixing apparatus in accordance with any one of the preceding claims, **characterized in that** the plate has a recess (66) in the region of the opening for the partial or also complete reception of the head of the anchorage element.

6. A bone fixing apparatus in accordance with any one of the preceding claims, **characterized in that** the head has an external thread (28) and the opening or a recess (66) of the opening has an internal thread (22) into which the head can be screwed.

7. A bone fixing apparatus in accordance with any one of the preceding claims, **characterized in that** a blocking element (30) is provided to block the head within the opening or within a recess (66) of the opening.

8. A bone fixing apparatus in accordance with claim 7, **characterized in that** the opening or the recess has an internal thread (22) and a closure element, in particular a closure screw (30) or a closure ring, is provided as the blocking element and has an external thread for the blocking of the head by screwing into the opening or into the recess.

9. A bone fixing apparatus in accordance with any one of the preceding claims, **characterized in that** the plate has a concave support surface in the region of the opening or of the recess of the opening facing the side of the plate facing the bone; and/in or that the head is designed spherically at the end facing the shaft such that the anchorage element is variable with respect to its angular position with respect to the plate before a fixing.

10. A bone fixing apparatus (10; 11) in accordance with any one of the preceding claims, **characterized in that** it is designed such that the fracture fixing plate (20; 21) can substantially be fixed without contact to the bone surface.

11. A bone fixing apparatus (10; 11) in accordance with any one of the preceding claims, **characterized in that** the fracture fixing plate (20; 21) is designed for arrangement in the epiphyseal and/or metaphyseal region of a bone, in particular of the proximal humerus.

12. A bone fixing apparatus in accordance with any one of the preceding claims, **characterized in that** the dimensions of the head and of the opening are adapted to one another such that the head cannot be passed through the opening.

13. A bone fixing apparatus in accordance with any one of the preceding claims, **characterized in that** the complete shaft of the anchorage element, but not the head of the anchorage element, can be passed through the opening.

14. A bone fixing apparatus in accordance with any one of the claims 2 to 13, **characterized in that** the element, in particular of pin-shape, is designed for the anchorage in the bone and the sleeve is designed for the fixing in the at least one opening of the fracture fixing plate.

15. A bone fixing apparatus in accordance with any one of the claims 2 to 13, **characterized in that** the sleeve is designed for the anchorage in the bone and the element, in particular of pin-shape, is designed for the fixing in the at least one opening of the fracture fixing plate.

## Revendications

1. Dispositif de fixation pour os (10 ; 11) destiné au traitement opératoire de fractures, incluant
une plaque de fixation de fracture (20 ; 21 ; 56) qui comporte au moins une ouverture (22), et
au moins un élément d'ancrage pour la fixation de la plaque de fixation de fracture sur un os, dans lequel l'élément d'ancrage comprend une tige ainsi qu'une tête (26, 27, 60), et dans lequel au moins une partie de la tige est susceptible d'être passée à travers l'ouverture, tandis que la tête est susceptible d'être fixée dans l'ouverture,
et dans lequel la tige est réalisée au moins en deux parties, avec une première partie (12, 13, 40, 44, 48 ; 52) sur laquelle est agencée la tête, et avec une seconde partie (14 ; 54) prévue pour l'ancrage dans l'os,
**caractérisé en ce que**, dans la situation du dispositif de fixation pour os fixé sur un os, la première partie et la seconde partie sont guidées en déplacement l'une par rapport à l'autre le long d'un axe.

2. Dispositif de fixation pour os selon la revendication 1, **caractérisé en ce que** l'élément d'ancrage inclut une douille (12, 13 ; 54) avec une zone de guidage intérieure et un élément (14, 40, 44, 48 ; 52) reçu dans celle-ci, en particulier en forme de tige, avec une zone de guidage extérieure, lesdites zones présentant chacune une section transversale constante, en particulier une section transversale de forme circulaire, en direction de l'axe, de telle façon que la zone de guidage extérieure est capable de coulisser en direction de l'axe en étant guidée linéairement dans la zone de guidage intérieure.

3. Dispositif de fixation pour os selon l'une des revendications précédentes,
**caractérisé en ce qu'**avant une fixation, la position angulaire (24) de l'élément d'ancrage par rapport à la plaque est susceptible d'être librement réglé à l'intérieur d'une plage prédéterminée.

4. Dispositif de fixation pour os selon l'une des revendications précédentes, **caractérisé en ce qu'**il est prévu des moyens (22, 28, 30) afin de relier la tête de l'élément d'ancrage avec la plaque.

5. Dispositif de fixation pour os selon l'une des revendications précédentes, **caractérisé en ce que** la plaque comporte, dans la région de l'ouverture, un renfoncement (66) pour recevoir partiellement ou même complètement la tête de l'élément d'ancrage.

6. Dispositif de fixation pour os selon l'une des revendications précédentes, **caractérisé en ce que** la tête comporte un pas de vis extérieur (28) et l'ouverture ou un renfoncement (66) de l'ouverture comporte un pas de vis intérieur (22) dans lequel la tête est susceptible d'être vissée.

7. Dispositif de fixation pour os selon l'une des revendications précédentes, **caractérisé en ce qu'**il est prévu un élément de blocage (30) afin de bloquer la tête à l'intérieur de l'ouverture ou d'un renfoncement (66) de l'ouverture.

8. Dispositif de fixation pour os selon la revendication 7, **caractérisé en ce que** l'ouverture ou le renfoncement comporte un pas de vis intérieur (22), et **en ce qu'**il est prévu à titre d'élément de blocage un élément d'obturation, en particulier une vis d'obturation (30) ou une bague d'obturation, avec un pas de vis extérieur pour bloquer la tête par vissage dans l'ouverture ou dans le renfoncement.

9. Dispositif de fixation pour os selon l'une des revendications précédentes, **caractérisé en ce que** la plaque comporte, dans la région de l'ouverture ou du renfoncement de l'ouverture, orientée vers le côté de la plaque tournée vers l'os, une surface d'appui concave et/ou la tête est réalisée en forme de tonneau à l'extrémité tournée vers la tige, de telle façon qu'avant une fixation l'élément d'ancrage est variable quant à sa situation angulaire par rapport à la plaque.

10. Dispositif de fixation pour os (10 ; 11) selon l'une des revendications précédentes, **caractérisé en ce qu'**il est réalisé de telle façon que la plaque de fixation de fracture (20 ; 21) est susceptible d'être fixée sensiblement sans contact par rapport à la surface de l'os.

11. Dispositif de fixation pour os (10 ; 11) selon l'une des revendications précédentes, **caractérisé en ce que** la plaque de fixation de fracture (20 ; 21) est réalisée pour être agencée dans la zone épiphysaire et/ou métaphysaire d'un os, en particulier de l'humérus proximal.

12. Dispositif de fixation pour os selon l'une des revendications précédentes, **caractérisé en ce que** les dimensions de la tête et de l'ouverture sont accordées les unes aux autres de telle façon que la tête n'est pas capable de passer à travers l'ouverture.

13. Dispositif de fixation pour os selon l'une des revendications précédentes, **caractérisé en ce que** la tige complète de l'élément d'ancrage est susceptible d'être passée à travers l'ouverture, mais pas la tête de l'élément d'ancrage.

14. Dispositif de fixation pour os selon l'une des revendications 2 à 13, **caractérisé en ce que** l'élément, en particulier en forme de tige, est réalisé en vue de l'ancrage dans l'os et la douille est réalisée en vue de la fixation dans ladite au moins une ouverture de la plaque de fixation de fracture.

15. Dispositif de fixation pour os selon l'une des revendications 2 à 13, **caractérisé en ce que** la douille est réalisée en vue de l'ancrage dans l'os et l'élément, en particulier en forme de tige, est réalisé en vue de la fixation dans ladite au moins une ouverture de la plaque de fixation de fracture.
